# EUROPEAN PATENT SPECIFICATION

(11) **EP 4 241 708 B1**
(45) Date of publication and mention of the grant of the patent: **19.08.2026**
(21) Application number: 22305267.1
(22) Date of filing: 09.03.2022
(51) Int. Cl.: A61B 17/42, A61B 17/12, A61B 17/30

(54) **HEMOSTATIC DEVICE**
BLUTSTILLENDE VORRICHTUNG
DISPOSITIF HÉMOSTATIQUE

(43) Date of publication of application: 13.09.2023
(73) Proprietor: Matria Health Technologies, 38400 Saint Martin d'Heres (FR)
(72) Inventor: URVOY, François, 38330 SAINT-NAZAIRE-LES-EYMES (FR)
(74) Representative: Regimbeau

(56) References cited:
- EP-A1- 0 141 589
- CN-A- 112 169 143
- CN-U- 208 822 926
- KR-A- 20010 002 164
- US-A1- 2018 078 283

## Description

### TECHNICAL FIELD

The present disclosure relates to a hemostatic device, in particular intended to stop or reduce hemorrhages in a body cavity such as a uterus.

### TECHNICAL BACKGROUND

After childbirth, the mother's uterus is generally subjected to contractions that allow to stop the bleeding caused by the detachment of the placenta.

However, in some cases, the uterus may suffer from atony, so that such contractions do not occur or are not sufficient to stop bleeding, thereby generating a risk of maternal hemorrhage.

Various hemostatic devices have been developed to promote the contraction of the uterus and help stopping or reducing the hemorrhage.

Document EP 3 248 624 teaches a hemostatic device for treating postpartum hemorrhage, comprising a flat flexible plate presenting a pair of opposite faces configured to be placed in contact with a wall of the uterus and an empty internal volume in fluidic connection with a vacuum pump. Each face comprises a plurality of holes such that when a negative pressure is created in the internal volume by the pump, the walls of the uterus are attracted by the respective face of the plate and held together, which mechanically shrinks the uterus, thereby compacting the muscles forming the wall of the uterus and constricting the blood vessels to stop bleeding. However, the size of the uterus may vary from one patient to another one. It may be difficult to introduce the hemostatic device in a little uterus like a partially retracted uterus. It may thus be necessary to provide different formats of the hemostatic device to allow the practitioner to select the one fitting best the patient's uterus. In addition, the size of the uterus also decreases as the uterus contracts. Thus, once the uterus has attained the size of the flexible plate, the hemostatic device may hinder further contraction of the uterus.

Document WO 2020/123525 teaches a hemostatic device for treating postpartum hemorrhage, comprising a flexible portion configured to be inserted into the uterus, a seal configured to close the opening of the uterus, and a vacuum pump in fluidic connection with holes provided in the flexible portion. When the uterus is sealed, the pump creates a negative pressure within the uterus via the holes in order to facilitate contractile movement of the uterine wall and vessel constriction. In this device, various means may be provided to protect the holes from occlusion by tissues. Because of the shape of this hemostatic device, when it is in a uterus, the area of the uterine wall which is in contact with the hemostatic device is limited. Consequently, the hemostatic effect of the contact between the hemostatic device and a bleeding area of the uterus is limited. Moreover, the space remaining between the uterine wall and the hemostatic device allows the vacuum to escape from the neck of the uterus. In order to reduce this depression, an inflatable balloon is required which makes the hemostatic device complicated to use. Furthermore, the pressure applied in the area of the neck of the uterus can be excessive while the area of the neck of uterus is fragile and not very extensible.

Document US 2018/078283 discloses an expandable device for manipulating tissues inside a body.

### SUMMARY OF THE DISCLOSURE

There remains a need for a hemostatic device that can be easily inserted into a body cavity such as the uterus and conform to the shape of the cavity, while providing an efficient stimulation of the muscles forming the wall of the cavity and constriction of the blood vessels, without hindering further contraction of the uterus over time.

Some embodiments relate to a hemostatic device comprising at least one chamber delimited by a membrane, each chamber being configured to be fluidically connected to a vacuum source, the membrane being configured to be placed so as to face, at least partially, a bleeding area of a body cavity, the membrane comprising a plurality of holes leading into the chamber and configured to induce a negative pressure in the body cavity when a negative pressure is applied in the chamber by the vacuum source, the induced negative pressure being configured so that the walls of the cavity are attracted to the membrane, wherein the membrane has at least two opposite faces, each face comprising a plurality of folds extending in at least one first direction.

According to advantageous and non-limiting features, taken alone or in any combination:
The folds form a plurality of successive peaks and valleys extending on either side of a normal plane, wherein each side of the folds is oriented at an angle lower than 45° with respect to the normal plane.

Each side of the folds is oriented at an angle lower than 30° with respect to the normal plane.

The valleys of the first face are arranged so as to face the valleys of the second face.

At least part of the plurality of holes are located on the peaks.

Each face of the membrane extends according to a plane.

Each face of the membrane further comprises a plurality of folds extending in a second direction substantially perpendicular to the first direction so as to form a plurality of polyhedrons with a base having at least four sides.

The opposite faces of the membrane are linked in a linking area.

The holes are arranged in the membrane according to a constant pattern.

The membrane comprises two lateral sides connecting the two faces of the membrane, at least part of the plurality of holes of the plurality of holes being located on the lateral sides.

The hemostatic device comprises a single chamber configured to be fluidically connected to a vacuum source.

Each face of the membrane is closed on itself.

The hemostatic device has a trapezoidal shape.

The hemostatic device comprises a base comprising a primary channel configured to be connected to a vacuum source and to secondary channels, the secondary channels being fluidically connected to the chamber.

The base comprises holes leading to secondary channels.

The membrane is made of a flexible biocompatible polymer.

The hemostatic device is configured to be inserted in a uterus.

### BRIEF DESCRIPTION OF THE FIGURES

Further features and advantages will be described in the following description, based on the appended drawings, in which:
- FIG. 1 is a side view of a hemostatic device according to a first embodiment;
- FIG. 2 shows a perspective view of a hemostatic device according to the first possible embodiment;
- FIGS. 3A, 3B schematically illustrate the retractation of the hemostatic device;
- FIG. 4 is a sectional view of the hemostatic device;
- FIGS. 5A, 5B, 6A, and 6B schematically illustrate enlarged views of a portion of a hemostatic device according to different embodiments;
- FIG. 7 is an enlarged sectional view of a portion of FIG. 4;
- FIG. 8 is a side view of a hemostatic device according to a second embodiment;
- FIG. 9 is an enlarged view of a portion of FIG. 8;
- FIG. 10 is a schematic sectional enlarged view of a portion of FIG. 8;
- FIG. 11 is a sectional view of a base of the hemostatic device;
- FIG. 12 schematically illustrates successive steps of operation of the hemostatic device;

For the sake of legibility of the drawings, some components of the hemostatic device may have been omitted. In addition, the drawings are not necessarily drawn to scale.

In the drawings, identical reference signs designate elements that are identical to each other or that fulfil the same function. Thus, when one element has been described in detail with reference to one figure, it may not be described in detail again with reference to another figure.

### DETAILED DESCRIPTION OF EMBODIMENTS

The invention relates to a hemostatic device 1 configured to be inserted into a body cavity, such as a uterus, comprising a bleeding area.

The hemostatic device 1 comprises a membrane 4 provided with a plurality of folds 8, such that the hemostatic device 1 can have a plurality of positions comprising an expanded position and retracted positions. The expanded position corresponds to the position of the hemostatic device 1 when it is at rest, i.e. when the membrane 4 is not submitted to any force that could retract it. A retracted position corresponds to a position of the hemostatic device 1 when the folds 8 are brought closer to each other in at least one direction which is substantially orthogonal to the extension direction of the folds 8.

The hemostatic device 1 can be inserted into the body cavity when it is in a retracted position; then, due to the elasticity of the membrane 4, the hemostatic device 1 moves back to its expanded position to substantially fit the body cavity. The wall of the body cavity is attracted to the hemostatic device 1 due to the negative pressure induced in the body cavity by a vacuum circuit arranged into the hemostatic device 1, which causes bleeding to stop. The hemostatic device 1 can be moved back to a retracted position to extract the hemostatic device 1 from the body cavity.

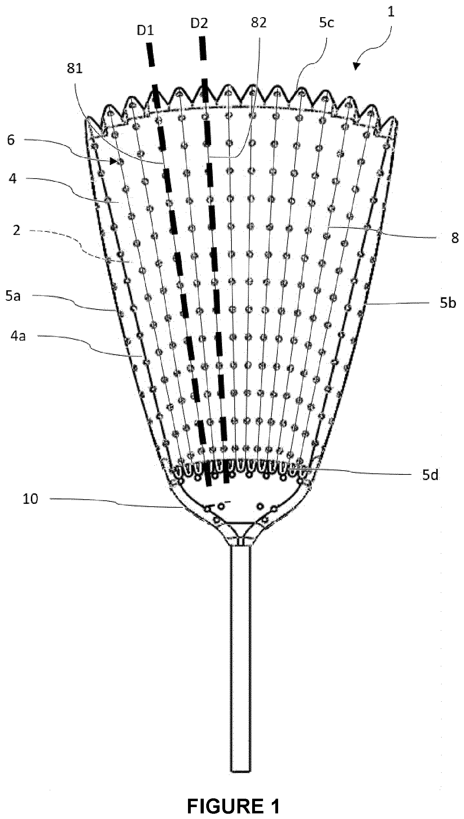

**FIG. 1** illustrates a side view of a hemostatic device 1 in a first embodiment. The hemostatic device 1 comprises at least one chamber 2 delimited by a membrane 4. The membrane 4 is configured to be placed so as to face, at least partially, a bleeding area of a body cavity. For example, the body cavity can be a uterus. Each chamber 2 is configured to be fluidically connected to a vacuum source. The membrane 4 comprises a plurality of holes 6 leading into the chamber 2 and configured to induce a negative pressure in the body cavity when a negative pressure is applied in the chamber 2 by the vacuum source, the induced negative pressure being configured so that the walls of the natural cavity are attracted to the membrane 4. This has a hemostasis effect. Moreover, the hemostatic device 1 can be retracted thanks to its geometrical structure.

In a particular embodiment, the hemostatic device 1 comprises a single chamber 2 adapted to be connected to the vacuum source. Thus, in the appended drawings and corresponding description, the hemostatic device 1 is illustrated with one chamber 2. However, it is understood that the hemostatic device can comprise more than one chamber 2, each chamber 2 being adapted to be connected to a common vacuum source or to distinct vacuum sources.

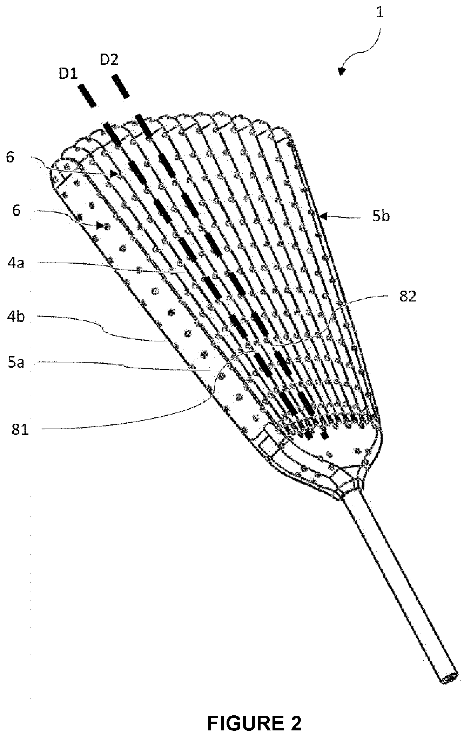

As illustrated in FIG. 2, the membrane 4 comprises two opposite faces 4a, 4b. The two opposite faces 4a, 4b are connected by two lateral sides 5a, 5b of the membrane 4. On an upper part of the hemostatic device 1, the two opposite faces are connected by an upper side 5c of the membrane 4. On a lower part of the hemostatic device 1, the lower part being opposite to the upper part, the two opposite faces are connected by a lower side 5d of the membrane 4.

Preferably, the hemostatic device 1 has a trapezoidal shape. The hemostatic device 1 can be seen as having the shape of a fan. Thus, in a side view like in **FIG. 1****,** the two lateral sides 5a, 5b flare out from the lower side 5d of the hemostatic device 1 to the upper side 5c.

The membrane 4 comprises a plurality of holes 6 leading into the chamber 2. When a negative pressure is applied in the chamber 2 by the vacuum source, the holes 6 induce a negative pressure in the body cavity. Thus, the walls of the body cavity are attracted to the membrane 4.

Preferably, the holes 6 of the membrane 4 are arranged according to a constant pattern. Indeed, in order to guarantee the vacuum distribution, there is preferably not one cm² of the membrane 4 which does not comprise a hole 6. Ideally, the holes 6 are equidistant from each other. Preferably, the holes 6 are arranged along lines.

The holes 6 are at least arranged on the two opposite faces 4a, 4b of the membrane 4. Preferably, as illustrated in **FIG.2****,** the two lateral sides 5a, 5b also comprise holes 6 inducing a better hemostasis effect since parts of the wall of the body cavity are attracted to the lateral sides 5a, 5b.

There are approximately between 100 and 1000 holes 6 on the membrane 4. The diameter of the holes 6 is in the range of one millimeter.

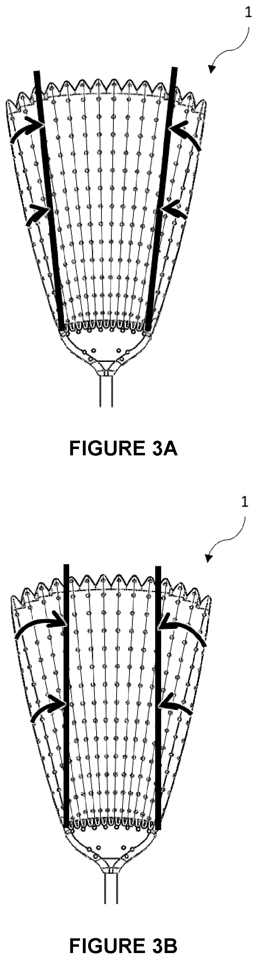

The two opposite faces 4a, 4b of the membrane 4 comprise a plurality of folds 8 extending in at least one direction. Preferably, the folds 8 extend in a linear direction, from the upper side to the lower side. Preferably, each fold 8 extends in a distinct direction. Thus, the folds 8 do not extend in parallel directions. Indeed, as said, in a certain embodiment, the hemostatic device 1 has the shape of a fan. Consequently, the folds 8 flare out from the lower side of the membrane 4 to the upper side. Thanks to these folds 8, the hemostatic device 1 can be retracted (like a fan). As illustrated is **FIGS. 1****,** **3A and 3B****,** it is understood that the hemostatic device 1 can have an expanded position (FIG.1) and several retracted positions (schematically illustrated in FIGS. 3A and 3B). The expanded position corresponds to the position of the hemostatic device 1 when it is "at rest", i.e. when it is not subjected to any force (manual or induced pressure) that could retract it. The retracted positions correspond to the positions of the hemostatic device 1 when it is retracted in at least one direction which is orthogonal to the extension direction of a fold 8. A fold 8 extends with respect to a direction of extension. For example, as illustrated in FIGS. 1 and 2, the fold 81 extends with respect to the extension direction D1 (schematically illustrated by a dotted line) and the fold 82 extends with respect to the extension direction D2 (schematically illustrated by a dotted line).

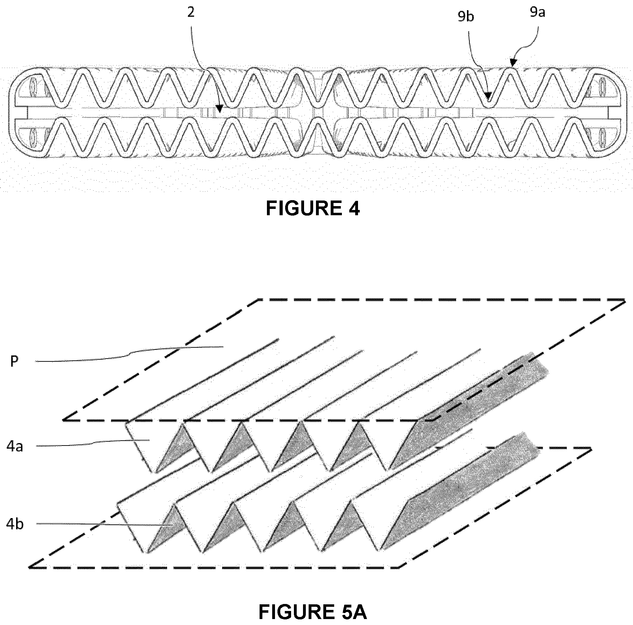

In a preferred embodiment, as illustrated in FIG. 4, the folds 8 form a plurality of successive peaks 9a and valleys 9b. As seen from the outside of the hemostatic device 1, the peaks 9a are protrusions and the valleys 9b are recesses extending between consecutive protrusions. The peaks 9a are closer than the valleys 9b to the wall of the body cavity when the hemostatic device 1 is into such body cavity. The folds 8 can be seen as the folds 8 of an accordion. The folds 8 comprise a plurality of sides 8a, 8b.

According to an embodiment illustrated in **FIGS. 4 and 5A****,** the two opposite faces 4a, 4b of the membrane 4 extend according to a plane. Otherwise said, all the peaks 9a of each face of the membrane 4 belong to a respective common plane P, both planes P being parallel to each other. The planes P are illustrated in dotted line in **FIG.** 5A.

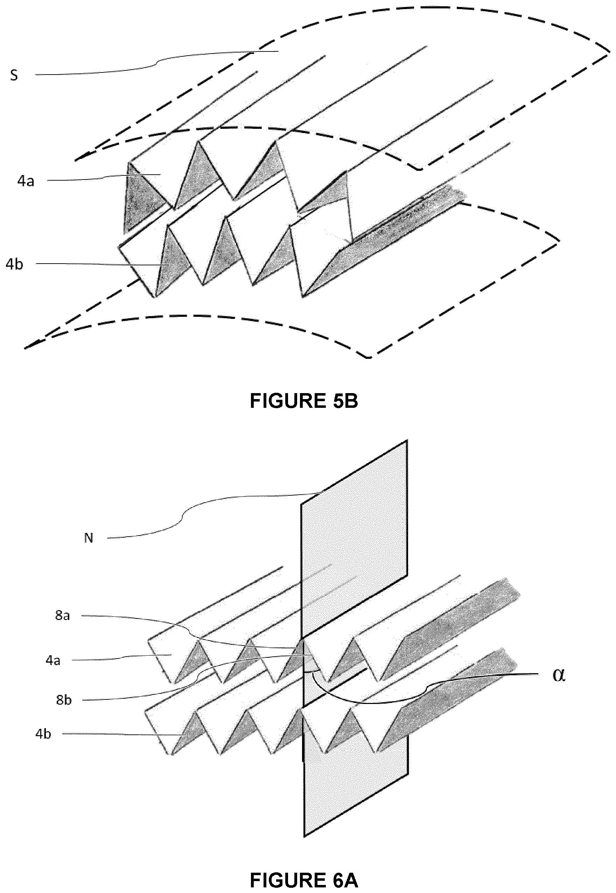

According to another embodiment illustrated in **FIG. 5B****,** the two opposite faces 4a, 4b of the membrane 4 extend according to a curved surface S. It is understood that this embodiment is quite similar to the previous one, the only difference being that the surface to which the peaks 9a of a respective face belong is curved, preferably with a convex shape or concave shape (depending on the face 4a or the face 4b considered). In FIG. 5B, the face 4a has a convex shape (meaning that it is rounded outward with respect to the chamber 2) and the face 4b has a concave shape (meaning that it is rounded inward with respect to the chamber 2). Both surfaces are substantially parallel to each other. The surfaces S are illustrated in dotted line in **FIG. 5B****.** This hemostatic device 1 has the advantage to better adapt to the shape of a body cavity such as a uterus.

According to yet another embodiment, the two opposite faces 4a, 4b of the membrane 4 are closed on themselves. Consequently, the two opposite faces 4a, 4b extend according to a respective curved surface closed on itself. This hemostatic device 1 has the advantage to better adapt to the shape of a body cavity such as a uterus.

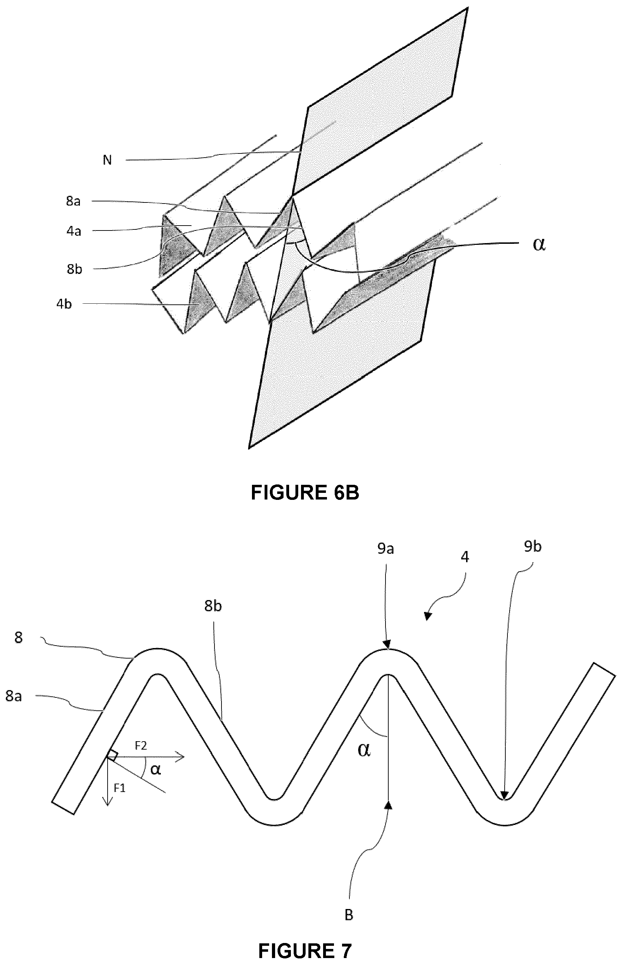

As illustrated in **FIGS. 6A****,** **6B and 7****,** each side 8a, 8b of the folds 8 is oriented at an angle α lower than 45° with respect to a normal plane N. It is understood that each side 8a, 8b of the folds 8 is oriented at an angle α lower than 45° with respect to a respective normal plane N such that each side 8a, 8b is associated with its own normal plane. Preferably, each side 8a, 8b of the folds 8 is oriented at an angle α lower than 30° with respect to the normal plane N. For each side 8a, 8b of the folds 8, the normal plane N extends according to the first direction of the fold 8, i.e. the direction in which the fold 8 extends, and according to a direction normal to the floor when the one of the opposite faces of the membrane 4 is on the floor such that the peak 9a comprising said side is on the floor. More precisely, the bisector B of the angle formed by a peak 9a (between two sides 8a, 8b of a peak) must extend in a normal direction of the floor.

In other words, for each peak 9a and each valley, the sides 8a, 8b of the peak 9a/valley 9b have the same length and the angle between the bisector B of the angle formed by the peak 9a/valley 9b (between two sides 8a, 8b of the peak 9a/valley 9b) and each side 8a, 8b of the peak 9a/valley 9b is lower than 45°, preferably lower than 30°

This geometrical structure prevents the hemostatic device 1 from being flattened because of the negative pressure applied into the hemostatic device 1.

Preferably, the holes 6 of the membrane 4 are arranged on the peaks 9a of the folds 8. When the hemostatic device 1 is in a body cavity, the peaks 9a are closer to the wall of the body cavity than the valleys 9b. Hence, the fact that holes 6 are arranged on the peaks 9a allows a better attraction of the wall to the membrane 4.

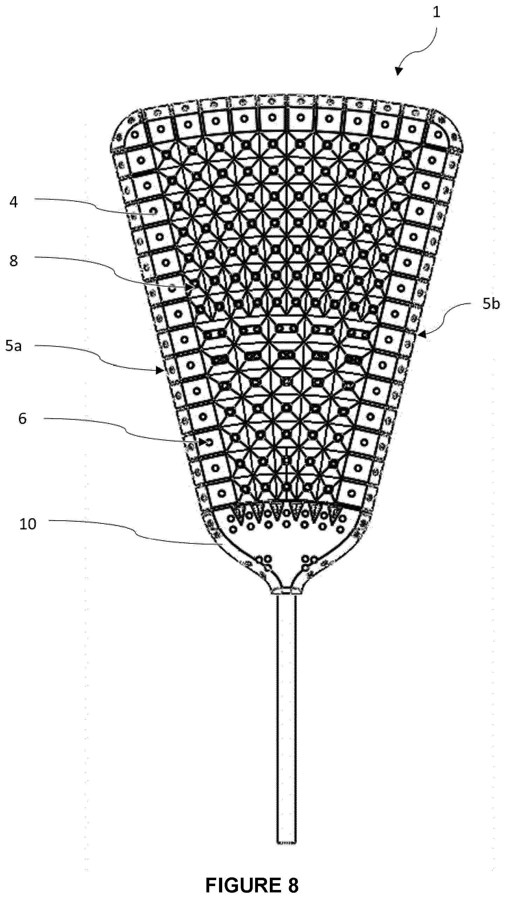

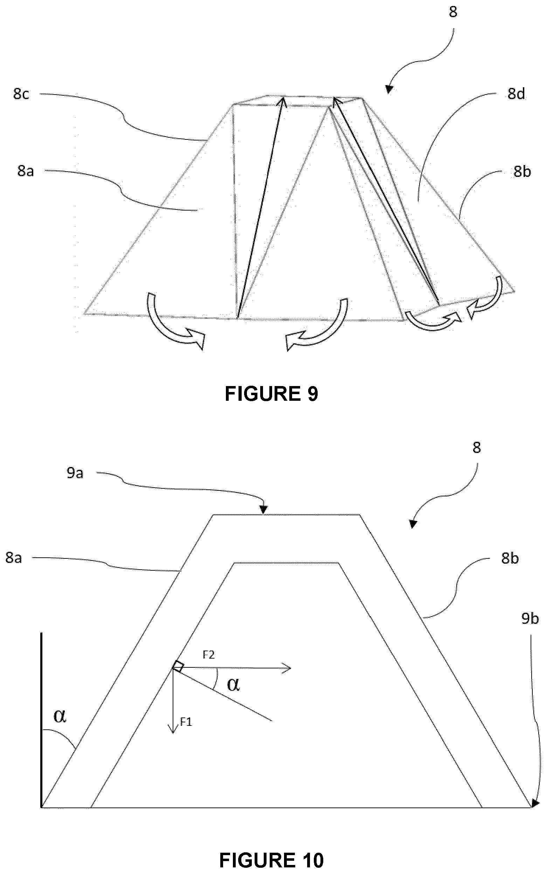

In a second embodiment illustrated in **FIG. 8****,** each opposite face 4a, 4b of the membrane 4 further comprises a plurality of folds 8 extending in a second direction substantially perpendicular to the first direction so as to form a plurality of polyhedrons with a base 10 having at least four sides. In this embodiment, the folds 8 form shapes more complex than peaks 9a and valleys 9b. In the illustrated example, the folds 8 form a plurality of pyramids having four sides 8a, 8b, 8c, 8d (see **FIG. 9****)** and a plurality of facets. Indeed, the base 10 of the pyramids illustrated have more than four sides (they have eight sides) and the pyramids have thirteen facets (an upper facet and three facets for each side 8a, 8b, 8c, 8d). The facet on the top of the pyramid preferably comprises at least one hole 6. This multi-facets structure facilitate the retraction of the hemostatic device 1 as shown in **FIG.9****.**

Even if the polyhedron described here is a pyramid, it is understood that the polyhedrons can be other polyhedrons. For example, it can be pyramids with less facets, parallelepipeds...

It is understood that the top facet of a polyhedron corresponds to a peak 9a in the first embodiment with peaks 9a and valleys 9b. The lateral faces of the polyhedron correspond to the sides 8a, 8b of the folds 8 in the first embodiment with peaks 9a and valleys 9b.

Here again, as illustrated in **FIG. 10****,** the folds 8 preferably respect the criteria according to which the angle α is lower than 45° and, in another preferred embodiment, lower than 30°. Thus, each side 8a, 8b, 8c, 8d of the folds 8 is oriented at an angle α lower than 45°, preferably lower than 30°, with respect to a normal plane N. As already explained, it is understood that each side 8a, 8b, 8c, 8d of the folds 8 is oriented at an angle α lower than 45°, preferably lower than 30°, with respect to a respective normal plane N such that each side 8a, 8b, 8c, 8d is associated with its own normal plane.

Preferably, the peaks 9a of the first opposite face 4a of the membrane 4 (and/or, in the embodiment with polyhedrons, the top facet of the polyhedron) face the peaks 9a of the second opposite face 4b of the membrane 4. Accordingly, the valleys 9b of the first opposite face 4a of the membrane 4 face the valleys 9b of the second opposite face 4b of the membrane 4. Consequently, the peaks 9a of the first opposite face 4a and the valley 9b of the second opposite face 4b cannot fit together such that the holes 6 of the first face cannot be blocked by the holes 6 of the second face 4b (and vice versa).

Furthermore, in a preferred embodiment, the opposite faces 4a, 4b of the membrane 4 are linked in a linking area so as to prevent the sliding of an opposite face 4a, 4b with respect to another. Indeed, if the faces 4a, 4b are allowed to slide, the peaks 9a of the first opposite face 4a and the valley 9b of the second opposite face 4b can fit together such that the holes 6 of the first face 4a can be blocked by the holes 6 of the second face 4b (and vice versa). This is highly unwanted. Thus, the faces 4a, 4b can be linked at some points inside the chamber 2 of the hemostatic device 1. For example, pillars can extend between the first opposite face 4a and of the second opposite face 4b. More precisely, pillars can extend between valleys 9b of the first opposite face 4a and valleys 9b of the second opposite face 4b.

Preferably, the hemostatic device 1 is made of a flexible biocompatible polymer such as silicone or other silicone-based elastomers, or thermoplastic elastomer (TPE) like thermoplastic polyurethane (TPU). The hemostatic device 1 can for example be made by moulding.

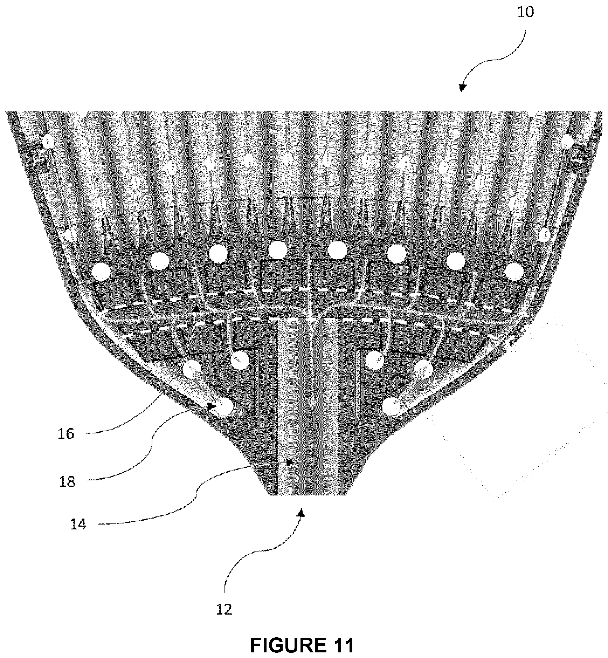

Preferably, to connect the hemostatic device 1 to the vacuum source, the hemostatic device 1 comprises a base 10 connected to the lower side 5d. **FIG. 11** schematically illustrates a sectional view of the base 10 and the circulation of fluid (such as blood) into the base 10 when the hemostatic device 1 is in use. Thus, the chamber 2 is fluidically connected to a vacuum source via the base 10 of the hemostatic device 1. The base 10 comprises an opening 12 configured to be connected to the vacuum source so that the chamber 2 is fluidically connected to the vacuum source. The opening 12 leads to a primary inner channel 14 of the base 10, the primary inner channel 14 leading to the chamber 2. Preferably, the primary inner channel 14 of the base 10 leads to secondary inner channels 16 of the base 10, the secondary inner channels 16 of the base 10 leading to the chamber 2.

The base 10 comprises a plurality of holes of the base 18 which are fluidically connected to the primary inner channel of the base 10. More precisely, the holes of the base 18 are fluidically connected to the secondary inner channels 16 of the base 10 which are fluidically connected to the primary inner channel 14 of the base 10.

The base 10 can be a distinct element which is assembled to the rest of the hemostatic device 1, i.e. the part made of the membrane 4 defining the chamber 2. In a preferred embodiment, the base 10 and the membrane 4 form a single piece and both are moulded in the same mould.

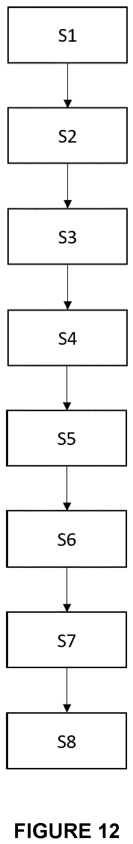

A complete workflow of a treatment using the hemostatic device 1 will be described with reference to **FIG. 12****.**

In step S1, the hemostatic device 1 is prepared by the medical staff. The preparation includes in particular unpacking the hemostatic device 1 from its package and connecting the hemostatic device 1 to the vacuum source. Preferably, the vacuum source is connected to the opening 12 of the base 10 such that the vacuum source is fluidically connected to the primary channel 14 of the base 10.

In step S2, the hemostatic device 1 is inserted into the body cavity, for example the uterus. When the hemostatic device 1 is inserted into the body cavity, it is preferably retracted. The folds 8 of the device allow the retraction of the device in same manner of the retraction of a fan. Thus, the hemostatic device 1 is retracted in at least one direction which is orthogonal to the extension direction of at least one fold 8 and the sides 8a, 8b of the folds 8 are tightened to each other. The retraction is typically performed manually by the medical staff.

In step S3, the medical staff stops the manual retraction of the hemostatic device 1. The hemostatic device 1 expands itself in the body cavity. In other words, the membrane 4 expands until it substantially fits the body cavity. By "fits the body cavity", it is meant that the membrane 4 substantially fits the shape and the size of the body cavity. By "substantially fit", it is meant that according to the size of the body cavity, the membrane 4 may not perfectly fit the internal wall of the body cavity, i.e. some areas of the wall of the body cavity may not be in contact with the internal wall of the body cavity. Indeed, for example, the size and shape of the uterus may vary for every woman. Moreover, given the fact that the membrane 4 is folded, it is obvious that parts of the membrane 4 (especially the valleys 9b) are not in contact with the wall of the body cavity. However, providing that the membrane 4 substantially fits the body cavity allows maintaining the effectiveness of the hemostatic device 1 regardless of such variations in size and shape. At the end of the step S3, the membrane 4 faces, at least partially, a bleeding area of the body cavity.

It can be noted that to adapt to very different sizes of body cavities (such as a normal uterus and a small uterus), several sizes like two sizes of the hemostatic device 1 can be provided. For a patient, the choice of the more adapted hemostatic device 1 is made by the medical staff.

The structure of the hemostatic device 1 is particularly efficient because it expands autonomously in the body cavity. No positive pressure is necessary to make the hemostatic device 1 expand accordingly to the body cavity. Without any intervention, the hemostatic device 1 is well disposed into the body cavity. The positioning of the hemostatic device 1 is easy and fast.

In step S4, the vacuum source is turned on so that a depression is applied into the body cavity. Indeed, it is reminded that the chamber 2 of the hemostatic device 1, which is fluidically connected to the vacuum source, is fluidically connected to the body cavity through the holes 6.

In the embodiment according to which the hemostatic device 1 comprises a base 10, the vacuum source applies a depression into the body cavity via the primary channel 14 which is fluidically connected to the secondary channels 16, the secondary channels 16 being fluidically connected to the chamber 2. It is reminded that the base 10 can also comprise holes of the base 18. Thus, the depression is also applied into the body cavity via the holes of the base 18.

As a result, the wall of the body cavity is attracted by the membrane 4.

The level of vacuum applied, i.e. the negative pressure applied in the body cavity, depends on the clinical case. In the case of a uterus, if the uterus is capable of contracting again quite autonomously, it only needs to be incited to contract again and a low negative pressure is sufficient, for example a negative pressure of 10 mbar. However, if the uterus is atonic, an important negative pressure is necessary, for example a negative pressure of 1000 mbar.

Step S5 is a first stabilization step, in which the vacuum is maintained. Hemostasis is initiated by a dual action of the hemostatic device 1: (1) direct contact between the wall of the body cavity and the membrane 4 (and optionally the base 10) of the hemostatic device 1 (promoting a so-called "contact hemostasis") and (2) compression of the wall of the body cavity and its internal structure by aspiration. Thanks to the fact that the holes 6 are arranged in a constant pattern, an even vacuum distribution can be ensured.

Moreover, during step S5, blood and bleeding tissues enter the chamber 2 via the holes 6. The blood and the bleeding tissues preferably circulate into the primary channel 14 and then into the secondary channels 16 of the base 10 to the opening 12. The blood and the bleeding tissues are evacuated via an atmospheric pump.

In step S6, the hemostatic device 1 retracts itself due to the vacuum applied in it. Indeed, once the wall of the body cavity is attracted to the holes 6 of the hemostatic device 1, the holes 6 are blocked by the wall and the vacuum is made into the chamber 2. Consequently, the hemostatic device 1 moves to a retracted position. Thanks to the specific structure of the membrane 4, the hemostatic device 1 retracts itself but is not flattened.

Indeed, the hemostatic device 1 is allowed to retract in a direction normal to the first direction, i.e. the folds 8 are tightened and are closer to each other. It is reminded that this retraction is useful because the hemostatic can adapt to the variations of size of the body cavity (the variations can be due to the natural size of the body cavity which is different from a person to another and/or due to the variation of the body cavity which, as the uterus, can contract itself over time). However, the flattening of the hemostatic device 1 would be detrimental to hemostasis effect because the holes 6 of a face of the membrane 4 would be blocked by the other opposite face due to the fact that the opposite faces 4a, 4b of the membrane 4 would be stuck to each other. This could hinder the application of the depression into the body cavity. Such a situation can be avoided thanks to the geometrical structure of the membrane 4. Indeed, the folds 8 are made so that, for each fold 8, a first force F1 which is directed with respect to a normal plane N of the fold 8 (i.e. a F1 is a force encouraging the hemostatic device 1 to flatten) is lower than a second force F2 which is directed orthogonally to the first force F1 and the extension direction of the fold 8 (i.e. F2 is a force encouraging the hemostatic device 1 to retract). The first force F1 and the second force F2 are represented on **FIGS. 7** **and** **10****.** As a conclusion, the hemostatic device 1 is ingeniously designed so as to prevent a flattening and to guarantee an efficient hemostasis.

During step S6, the body cavity size decreases. The reduction of the size of the natural body cavity promotes the contraction of the muscles. These contractions compress the vessels and stop the bleeding permanently.

In step S7, the negative pressure is stopped, i.e. the vacuum source is turned off.

In step S8, the hemostatic device 1 is extracted from the body cavity. The extraction is typically performed manually by the medical staff who retracts manually the hemostatic device 1 to extract it. The medical procedure ends, after a minimal treatment time of 10 minutes for example, depending on the specific protocol.

## Claims

1. Hemostatic device (1) comprising at least one chamber (2) delimited by a membrane (4), each chamber (2) being configured to be fluidically connected to a vacuum source, the membrane (4) being configured to be placed so as to face, at least partially, a bleeding area of a body cavity, the membrane (4) comprising a plurality of holes (6) leading into the chamber (2) and configured to induce a negative pressure in the body cavity when a negative pressure is applied in the chamber (2) by the vacuum source, the induced negative pressure being configured so that the walls of the cavity are attracted to the membrane (4),
wherein the membrane (4) has at least two opposite faces (4a, 4b),
**characterized in that** each face comprises a plurality of folds (8) extending in at least one first direction.

2. Hemostatic device (1) according to claim 1, wherein the folds (8) form a plurality of successive peaks (9a) and valleys (9b) extending on either side of a normal plane, wherein each side (8a, 8b, 8c, 8d) of the folds (8) is oriented at an angle lower than 45° with respect to the normal plane.

3. Hemostatic device (1) according to claim 2, wherein each side (8a, 8b, 8c, 8d) of the folds (8) is oriented at an angle lower than 30° with respect to the normal plane.

4. Hemostatic device (1) according to any one of claims 2 or 3, wherein the valleys (9b) of the first face (4a) are arranged so as to face the valleys (9b) of the second face (4b).

5. Hemostatic device (1) according to any one of claims 2 to 4, wherein at least part of the plurality of holes (6) are located on the peaks (9a).

6. Hemostatic device (1) according to any one of claims 1 to 5, wherein each face (4a, 4b) of the membrane (4) extends according to a plane.

7. Hemostatic device (1) according to any one of claims 1 to 6, wherein each face (4a, 4b) of the membrane (4) further comprises a plurality of folds (8) extending in a second direction substantially perpendicular to the first direction so as to form a plurality of polyhedrons with a base having at least four sides.

8. Hemostatic device (1) according to any one of claims 1 to 7, wherein the opposite faces (4a, 4b) of the membrane (4) are linked in a linking area.

9. Hemostatic device (1) according to any one of claims 1 to 8, wherein the holes (6) are arranged in the membrane (4) according to a constant pattern.

10. Hemostatic device (1) according to any one of claims 1 to 9, wherein the membrane (4) comprises two lateral sides (5a, 5b) connecting the two faces (4a, 4b) of the membrane (4), at least part of the plurality of holes (6) of the plurality of holes (6) being located on the lateral sides (5a, 5b).

11. Hemostatic device (1) according to any one of claims 1 to 10, comprising a single chamber (2) configured to be fluidically connected to a vacuum source.

12. Hemostatic device (1) according to any one of claims 1 to 11, wherein each face of the membrane (4) is closed on itself.

13. Hemostatic device (1) according to any one of claims 1 to 11, wherein the hemostatic device (1) has a trapezoidal shape.

14. Hemostatic device (1) according to any one of claims 1 to 13, comprising a base (10) comprising a primary channel (14) configured to be connected to a vacuum source and to secondary channels (16), the secondary channels (16) being fluidically connected to the chamber (2).

15. Hemostatic device (1) according to claim 14, wherein the base (10) comprises holes (6) leading to secondary channels (16).

16. Hemostatic device (1) according to any one of claims 1 to 15, wherein the membrane (4) is made of a flexible biocompatible polymer.

17. Hemostatic device (1) according to any one of claims 1 to 16, configured to be inserted in a uterus.

## Patentansprüche

1. Blutstillende Vorrichtung (1), die mindestens eine Kammer (2) umfasst, die von einer Membran (4) abgegrenzt ist, wobei jede Kammer (2) dazu ausgestaltet ist, fluidisch mit einer Vakuumquelle verbunden zu werden, wobei die Membran (4) dazu ausgestaltet ist, platziert zu werden, um einem blutenden Bereich einer Körperhöhle zumindest teilweise zugewandt zu sein, wobei die Membran (4) eine Vielzahl von Löchern (6) umfasst, die in die Kammer (2) führen und dazu ausgestaltet sind, einen Unterdruck in der Körperhöhle herbeizuführen, wenn ein Unterdruck in der Kammer (2) durch die Vakuumquelle angewandt wird, wobei der herbeigeführte Unterdruck derart ausgestaltet ist, dass die Wände der Höhle an die Membran (4) angezogen werden,
wobei die Membran (4) mindestens zwei entgegengesetzte Flächen (4a, 4b) aufweist, **dadurch gekennzeichnet, dass** jede Fläche eine Vielzahl von Falten (8) umfasst, die sich in mindestens einer ersten Richtung erstrecken.

2. Blutstillende Vorrichtung (1) nach Anspruch 1, wobei die Falten (8) eine Vielzahl von aufeinanderfolgenden Spitzen (9a) und Tälern (9b) bilden, die sich auf beiden Seiten einer Normalebene erstrecken, wobei jede Flanke (8a, 8b, 8c, 8d) der Falten (8) in einem Winkel ausgerichtet ist, der in Bezug auf die Normalebene kleiner als 45° ist.

3. Blutstillende Vorrichtung (1) nach Anspruch 2, wobei jede Flanke (8a, 8b, 8c, 8d) der Falten (8) in einem Winkel von kleiner als 30° in Bezug auf die Normalebene ausgerichtet ist.

4. Blutstillende Vorrichtung (1) nach einem der Ansprüche 2 oder 3, wobei die Täler (9b) der ersten Fläche (4a) derart angeordnet sind, dass sie den Tälern (9b) der zweiten Fläche (4b) zugewandt sind.

5. Blutstillende Vorrichtung (1) nach einem der Ansprüche 2 bis 4, wobei zumindest ein Teil der Vielzahl von Löchern (6) sich auf den Spitzen (9a) befindet.

6. Blutstillende Vorrichtung (1) nach einem der Ansprüche 1 bis 5, wobei jede Fläche (4a, 4b) der Membran (4) sich entlang einer Ebene erstreckt.

7. Blutstillende Vorrichtung (1) nach einem der Ansprüche 1 bis 6, wobei jede Fläche (4a, 4b) der Membran (4) ferner eine Vielzahl von Falten (8) umfasst, die sich in einer zweiten Richtung im Wesentlichen senkrecht zur ersten Richtung erstrecken, um eine Vielzahl von Vielflächnern mit einer Basis zu bilden, die mindestens vier Seiten aufweist.

8. Blutstillende Vorrichtung (1) nach einem der Ansprüche 1 bis 7, wobei die entgegengesetzten Flächen (4a, 4b) der Membran (4) in einem Verbindungsbereich verbunden sind.

9. Blutstillende Vorrichtung (1) nach einem der Ansprüche 1 bis 8, wobei die Löcher (6) in der Membran (4) gemäß einem konstanten Muster angeordnet sind.

10. Blutstillende Vorrichtung (1) nach einem der Ansprüche 1 bis 9, wobei die Membran (4) zwei Längsseiten (5a, 5b) umfasst, die die zwei Flächen (4a, 4b) der Membran (4) verbinden, wobei zumindest ein Teil der Vielzahl von Löchern (6) sich auf den Längsseiten (5a, 5b) befindet.

11. Blutstillende Vorrichtung (1) nach einem der Ansprüche 1 bis 10, die eine einzelne Kammer (2) umfasst, die dazu ausgestaltet ist, fluidisch mit einer Vakuumquelle verbunden zu sein.

12. Blutstillende Vorrichtung (1) nach einem der Ansprüche 1 bis 11, wobei jede Fläche der Membran (4) auf sich selbst geschlossen ist.

13. Blutstillende Vorrichtung (1) nach einem der Ansprüche 1 bis 11, wobei die blutstillende Vorrichtung (1) eine trapezförmige Form aufweist.

14. Blutstillende Vorrichtung (1) nach einem der Ansprüche 1 bis 13, die eine Basis (10) umfasst, die einen Hauptkanal (14) umfasst, der dazu ausgestaltet ist, mit einer Vakuumquelle und mit Nebenkanälen (16) verbunden zu werden, wobei die Nebenkanäle (16) fluidisch mit der Kammer (2) verbunden sind.

15. Blutstillende Vorrichtung (1) nach Anspruch 14, wobei die Basis (10) Löcher (6) umfasst, die zu Nebenkanälen (16) führen.

16. Blutstillende Vorrichtung (1) nach einem der Ansprüche 1 bis 15, wobei die Membran (4) aus einem biegsamen biokompatiblen Polymer besteht.

17. Blutstillende Vorrichtung (1) nach einem der Ansprüche 1 bis 16, die dazu ausgestaltet ist, in einen Uterus eingeführt zu werden.

## Revendications

1. Dispositif hémostatique (1) comprenant au moins une chambre (2) délimitée par une membrane (4), chaque chambre (2) étant configurée pour être reliée fluidiquement à une source de vide, la membrane (4) étant configurée pour être placée de manière à faire face, au moins partiellement, à une zone de saignement d'une cavité corporelle, la membrane (4) comportant une pluralité de trous (6) débouchant dans la chambre (2) et configurés pour induire une pression négative dans la cavité corporelle lorsqu'une pression négative est appliquée dans la chambre (2) par la source de vide, la pression négative induite étant configurée de telle sorte que les parois de la cavité soient attirées vers la membrane (4),
dans lequel la membrane (4) présente au moins deux faces opposées (4a, 4b), **caractérisé en ce que** chaque face comprend une pluralité de plis (8) s'étendant dans au moins une première direction.

2. Dispositif hémostatique (1) selon la revendication 1, dans lequel les plis (8) forment une pluralité de crêtes (9a) et de creux (9b) successifs s'étendant de part et d'autre d'un plan normal, chaque flanc (8a, 8b, 8c, 8d) des plis (8) étant orientée selon un angle inférieur à 45° par rapport au plan normal.

3. Dispositif hémostatique (1) selon la revendication 2, dans lequel chaque flanc (8a, 8b, 8c, 8d) des plis (8) est orienté selon un angle inférieur à 30° par rapport au plan normal.

4. Dispositif hémostatique (1) selon l'une quelconque des revendications 2 ou 3, dans lequel les creux (9b) de la première face (4a) sont agencés de manière à faire face aux creux (9b) de la deuxième face (4b).

5. Dispositif hémostatique (1) selon l'une quelconque des revendications 2 à 4, dans lequel au moins une partie de la pluralité de trous (6) est située sur les crêtes (9a).

6. Dispositif hémostatique (1) selon l'une quelconque des revendications 1 à 5, dans lequel chaque face (4a, 4b) de la membrane (4) s'étend selon un plan.

7. Dispositif hémostatique (1) selon l'une quelconque des revendications 1 à 6, dans lequel chaque face (4a, 4b) de la membrane (4) comprend en outre une pluralité de plis (8) s'étendant dans une deuxième direction sensiblement perpendiculaire à la première direction de manière à former une pluralité de polyèdres dont la base comporte au moins quatre côtés.

8. Dispositif hémostatique (1) selon l'une quelconque des revendications 1 à 7, dans lequel les faces opposées (4a, 4b) de la membrane (4) sont reliées au niveau d'une zone de liaison.

9. Dispositif hémostatique (1) selon l'une quelconque des revendications 1 à 8, dans lequel les trous (6) sont disposés dans la membrane (4) selon un motif constant.

10. Dispositif hémostatique (1) selon l'une quelconque des revendications 1 à 9, dans lequel la membrane (4) comprend deux côtés latéraux (5a, 5b) reliant les deux faces (4a, 4b) de la membrane (4), au moins une partie de la pluralité de trous (6) étant située sur les côtés latéraux (5a, 5b).

11. Dispositif hémostatique (1) selon l'une quelconque des revendications 1 à 10, comprenant une chambre unique (2) configurée pour être reliée de manière fluidique à une source de vide.

12. Dispositif hémostatique (1) selon l'une quelconque des revendications 1 à 11, dans lequel chaque face de la membrane (4) est fermée sur elle-même.

13. Dispositif hémostatique (1) selon l'une quelconque des revendications 1 à 11, dans lequel le dispositif hémostatique (1) présente une forme trapézoïdale.

14. Dispositif hémostatique (1) selon l'une quelconque des revendications 1 à 13, comprenant une base (10) comportant un canal principal (14) configuré pour être relié à une source de vide et à des canaux secondaires (16), les canaux secondaires (16) étant reliés de manière fluidique à la chambre (2).

15. Dispositif hémostatique (1) selon la revendication 14, dans lequel la base (10) comporte des trous (6) débouchant dans les canaux secondaires (16).

16. Dispositif hémostatique (1) selon l'une quelconque des revendications 1 à 15, dans lequel la membrane (4) est réalisée en un polymère souple biocompatible.

17. Dispositif hémostatique (1) selon l'une quelconque des revendications 1 à 16, configuré pour être inséré dans l'utérus.
